# EUROPEAN PATENT APPLICATION

(11) **EP 3 144 029 A1**
(43) Date of publication of application: **22.03.2017**
(21) Application number: 15191374.6
(22) Date of filing: 26.10.2015
(51) Int. Cl.: A61M 37/00

(54) **NON- RESILIENT TATTOOING NEEDLE AND TATTOOING DEVICE**

(30) Priority: 15.09.2015 CN 201510585196
(71) Applicant: E-M medical treatment and electron (suzhou) co., ltd, Jiangsu (CN)
(72) Inventor: LEE, BOB OSCAR, Suzho (CN)
(74) Representative: KIPA AB

(57) **Abstract**

A non-resilient tattoing needle comprises a barrel 1, a needle body 2, a needle base 3, a first connecting rod 4 and a first connecting part 5. The first connecting part of the present invention reciprocates under an external force and thus drives the needle body, via the first connecting rod and the needle base, to reciprocate linearly in the barrel. The needle of the present invention is driven by an external force directly which enables the needle to retract quickly. A tattooing device comprising an electric motor, a handle housing, a second connecting rod and an aforementioned tattooing needle. When pushing out the needle body, the electric motor does not need to work against resilient forces. Therefore, both the current and power of the electric motor are kept stable which thereby greatly prolongs the service life thereof.

## Description

### Technical Field

The present invention generally relates to medical instruments and particularly to a non- resilient tattooing needle and a tattooing device.

### Background

In today's society, the body art industry develops increasingly fast. People would like to beautify themselves by undergoing tattooing, permanent makeup, etc. One-time usage tattooing needles and tattooing devices are all needed in these processes.

Usually, a conventional tattooing device is configured such that the needle body used therein can automatically reciprocate to facilitate tattooing. A common needle employs a spring, a rubber band or a rubber ring to provide resilient force so the needle will always be forced to a retracted position. When in use, the needle body extends forward due to a driving force (such as a motor or solenoids), during which the spring, the rubber band or the rubber ring is stretched to accumulate resilient force. When the driving of the electric motor is removed, the needle will retract by way of the resilient force.

However, using resilient force as the retracting force has many disadvantages. First, the needle retracts by way of resilient force which requires certain deformation to work. Thus, the retracting speed of the needle gradually decreases and certain delay will be caused so that the needle cannot retract quickly. Second, the spring, the rubber band or the rubber ring may lose resilience after long-term use so that the needle cannot retract at the correct speed. Third, when driving the needle, the electric motor needs to work against resilient force. As the resilient force becomes higher, the power needed by the electric motor also becomes increasingly larger. Clearly, an electric motor can be easily damaged and its service life will be shortened due to the wear and tear of the added stress put on it from the resilient force.

### Summary of the Invention

One objective of the present invention is to provide a non- resilient tattooing needle with a simple structure that is free from the problems associated with the resilient forces of a spring, a rubber band or a rubber ring. Another objective of the present invention is to provide a tattooing device that is capable of retracting the needle quickly and effectively extending the service life of the electric motor.

According to one aspect of the present invention, a non- resilient tattooing needle is provided which comprises of a barrel, a needle body, a needle base, a first connecting rod and a first connecting part. The needle body is disposed inside the barrel and the needle base is configured for fixing the needle body. The first connecting rod is configured for connecting with the needle base and is arranged along the same axis with the needle body. The first connecting part is disposed on the top of the first connecting rod. The first connecting part of the present invention can reciprocate under an external force and thus drives the needle body, via the first connecting rod and the needle base, to reciprocate linearly in the barrel.

The advantageous effect of the above solution is that the needle does not employ any spring, rubber band or rubber ring to provide resilient force but it is just driven by an external force directly. The external force drives the first connecting part, which in turn drives the needle body, via the first connecting rod and the needle base, to reciprocate linearly in the barrel.

The retraction of the needle of the present invention does not rely on resilient force but only relies on the driving of the external force. Therefore, retraction delay is avoided. By quickly changing the external force, a quick retraction of the needle can be realized. In addition, the present invention does not employ any spring, rubber band or rubber ring to provide resilient force, such a problem is avoided that the spring, the rubber band or the rubber ring may lose resilience after long-term use which may cause the needle to retract improperly.

In some embodiments, a sliding block is connected in-between the first connecting rod and the needle base. The barrel includes a sliding barrel to cooperate with the sliding block. And the sliding block is capable of reciprocating linearly within the sliding barrel.

The advantageous effect of the above solution is that a guiding function can be realized by cooperation between the sliding block and the sliding barrel so that the first connecting rod can only reciprocate linearly within the sliding barrel. By driving the needle body to only reciprocate linearly with the needle base, shaking and side-to-side motion can be avoided so that subsequent tattooing can be performed conveniently and correctly.

In some embodiments, a position limiting ring is provided at the bottom of the sliding barrel. An end cover is provided at the top of the sliding barrel and it is fitted over the first connecting rod and located between the sliding block and the first connecting part. The sliding block reciprocates linearly between the position limiting ring and the end cover.

The advantageous effect of the above solution is that by cooperation between the position limiting ring and the end cover, the sliding block can only reciprocate linearly between the end cover and position limiting ring. When the needle body is extended, the extending length of the needle body is restricted by the position limiting ring, which effectively prevents receivers from being tattooed too deep.

In some embodiments, the barrel further includes an ink chamber and a needle nozzle. On sidewall of the ink chamber are provided with an injection hole and antislip stripes. The ink chamber is connected with the sliding barrel. The needle nozzle has an approximately tapered shape and includes a tapered mouth and a tapered tip. The tapered mouth is connected with the ink chamber. The needle body reciprocates linearly inside the tapered tip and is capable of extending beyond or retracting within the tapered tip.

The advantageous effect of the above solution is that the antislip stripes can facilitate holding operations of users. The tapered shape of the needle nozzle can restrict the movement range of the needle body thereby preventing substantial shaking and side-to-side movement thereof and facilitating subsequent tattooing operations.

In some incidents, the needle nozzle is made of suitable transparent materials. The advantageous effect of the above solution is that a viewer can see the internal conditions of the needle nozzle conveniently, such as the ink level in the needle nozzle and the extending and retracting conditions of the needle body, thereby preventing jamming of the needle body.

According to another aspect of the present invention, a tattooing device is provided which comprises of an electric motor, a handle housing, a second connecting rod and an aforementioned tattooing needle. The electric motor is located at the top of the handle housing. The second connecting rod is arranged within the handle housing. The handle housing is to be connected with the barrel. One end of the second connecting rod is driven by the electric motor and the other end thereof is provided with a second connecting part that is to be rigidly connected with the first connecting part. The electric motor drives the second connecting rod to reciprocate linearly in the handle housing. The second connecting rod drives the first connecting rod to move. And, the first connecting rod drives the needle body to reciprocate linearly in the barrel.

The advantageous effect of the above solution is that the needle does not employ any spring, rubber band or rubber ring to provide resilient force but it is just driven by an electric motor directly. The electric motor drives the second connecting rod to reciprocate linearly in the handle housing, the second connecting rod drives the first connecting rod to move, and the first connecting rod in turn drives the needle body to reciprocate linearly in the barrel. Because the second and first connecting parts are rigidly connected, retraction of the needle of the present invention is driven by the electric motor directly, thereby avoiding a delayed retraction and realizing instant retraction. Further, because the extending and retraction of the needle of the present invention are driven by the electric motor directly, when pushing out the needle body, the electric motor does not need to work against resilient forces. Therefore, both the current and power of the electric motor are kept stable and this thereby greatly prolongs the service life thereof.

In some embodiments, the first connecting part and/or the second connecting part is/are a magnet/magnets. Other options include connecting the first and second connecting parts by hooking, clamping or buckling without magnets, which however renders the structure complex. A preferred connecting manner is by magnetic connection which can be realized by using a magnet as one of the first and second connecting parts or using magnets as both of them. Usually, the needle is a disposable member. To save cost, the second connecting part therefore is normally selected as a magnet and the first connecting part is selected from a material that can be easily attracted by a magnet (such as iron). A similar effect can be realized by designing the first and second connecting parts to be able to connect via a hooking, clamping or buckling.

In some embodiments, an output shaft of the electric motor is arranged perpendicular to the second connecting rod and it is connected with an eccentric shaft. The eccentric shaft rotates circumferentially about the output shaft of the electric motor as the axis and it is connected with the second connecting rod. When rotating circumferentially, the eccentric shaft drives the second connecting rod to reciprocate linearly in the handle housing.

The advantageous effect of the above solution is that, by rotation of the electric motor and the provision of the eccentric shaft, the second connecting rod can be driven to reciprocate linearly in the handle housing.

In some embodiments, the top of the second connecting rod is provided with a connecting ring equipped with a rubber ring and the eccentric shaft is fitted in the rubber ring.

The advantageous effect of the above solution is that, transmission of movement of the electric motor and the second connecting rod can be realized through cooperation among the eccentric shaft, the rubber ring and the connecting ring. Rotation of the electric motor can drive the second connecting rod to reciprocate linearly in the handle housing and the rubber ring can prevent abrasion of the connecting ring and it can be replaced when it is worn.

In some embodiments, on outer wall of the sliding barrel of the barrel there provided with two or more clamping edges usually disposed symmetrically. One side of the clamping edges is a connecting area and the other side thereof is a protruding part. The connecting area is in smooth connection with the external surface of the sliding barrel and a height difference is pre-set between the protruding part and the surface of the outer wall of the sliding barrel. And, one end of the handle housing connecting the barrel is of an elliptical shape.

The advantageous effect of the above solution is that, when connecting the sliding barrel of the barrel with the handle housing, it can be inserted into the handle housing in accordance with the long axis of the ellipse-shaped handle housing. Then, the sliding barrel is rotated in a direction from the protruding part towards the connecting area until the clamping edges are in close contact with the inner wall of the elliptical-shaped handle housing along its short axis direction. In this manner, a firm contact between the sliding barrel and the handle housing is achieved.

### Brief Description of the Drawings

Figure 1 is a schematic drawing of a non-resilient tattooing needle according to an embodiment of the present invention;
Figure 2 is a schematic drawing of a non-resilient tattooing needle according to an embodiment of the present invention in its retracted state;
Figure 3 is a schematic drawing of a non-resilient tattooing needle according to an embodiment of the present invention in its extended state;
Figure 4 is a schematic drawing of a non-resilient tattooing needle according to an embodiment of the present invention, in which the needle body, the needle base and the first connecting rod are assembled;
Figure 5 is a schematic drawing of a tattooing device according to an embodiment of the present invention;
Figure 6 is a schematic and partially exploded view of a tattooing device according to an embodiment of the present invention;
Figure 7 is a schematic and full exploded view of a tattooing device according to an embodiment of the present invention;
Figure 8 is a schematic drawing of part of a tattooing device according to an embodiment of the present invention;
Figure 9 is a schematic drawing of an electric motor according to an embodiment of the present invention in a connected state;
Figure 10 is a schematic drawing showing a situation when the sliding barrel of a tattooing device according to an embodiment of the present invention is inserted into the handle housing; and
Figure 11 is a schematic drawing showing a situation when the sliding barrel and the handle housing of a tattooing device according to an embodiment of the present invention are connected and rotated.

### Embodiments for Carrying Out the Invention

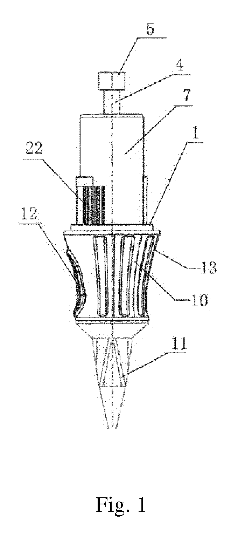

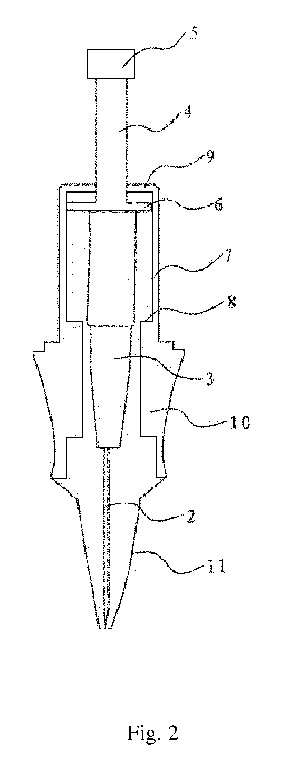

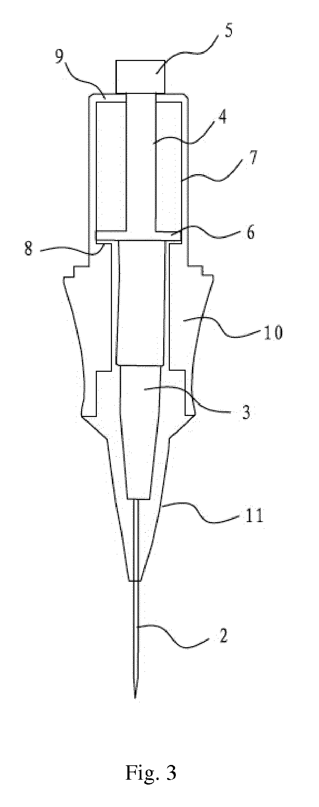

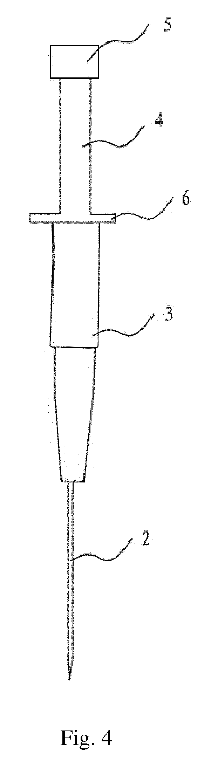

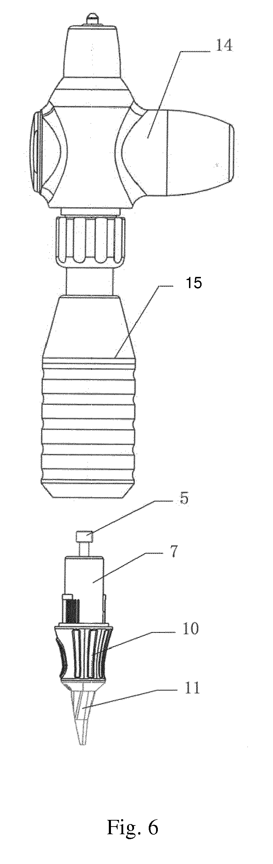

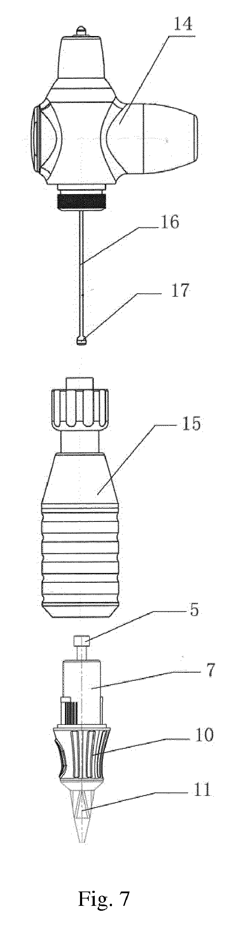

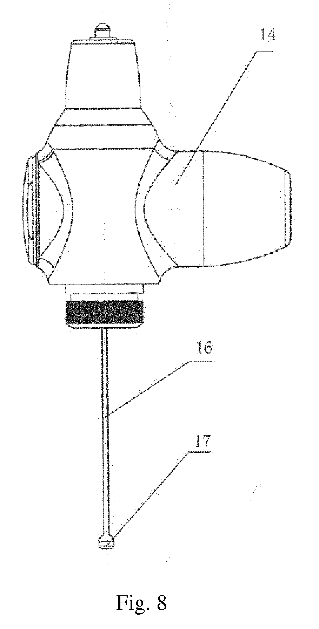

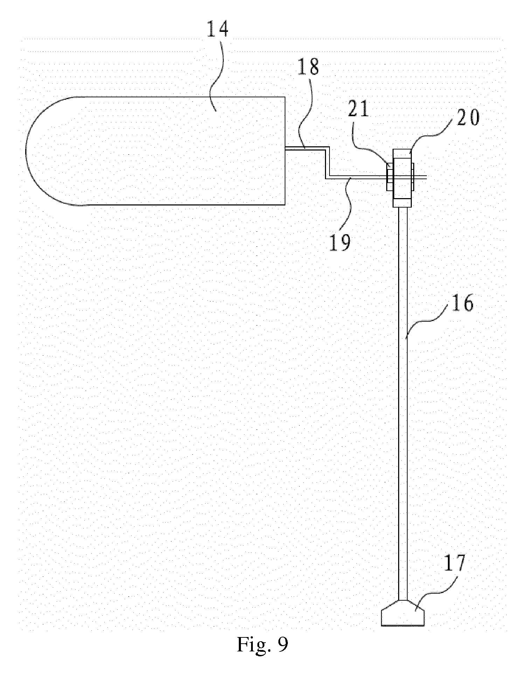

The present invention will be described in detail with reference to Figures 1 to 11.

Figures 1 to 4 are schematic drawings indicating a non- resilient tattooing needle according to an embodiment of the present invention. As shown in these figures, a non-resilient tattooing needle is provided which comprises: a barrel 1, a needle body 2 disposed inside the barrel 1, a needle base 3 for fixing/mounting the needle body 2, a first connecting rod 4 for connecting with the needle base 3 and arranged along the same axis with the needle body 2, and a first connecting part 5 disposed at the top of the first connecting rod 4.

The first connecting part 5 of the present invention reciprocates directly under an external force and thus drives the needle body 2, via the first connecting rod 4 and the needle base 3, to reciprocate linearly in the barrel 1.

The needle of the present invention does not employ any spring, rubber band or rubber ring to provide resilient force but it is merely driven by the external force directly. The external force drives the first connecting part 5, which in turn drives the needle body 2, via the first connecting rod 4 and the needle base 3, to reciprocate linearly in the barrel 1.

Because retraction of the needle of the present invention does not rely on resilient force but just rely on driving of external force(s), delay in the retraction could be avoided. By quickly changing the external force, a prompt retraction of the needle can be realized. In addition, the present invention does not employ any spring, rubber band or rubber ring to provide resilient force, such a problem can be avoided that the spring, the rubber band or the rubber ring may lose resilience after long-term use which would cause the needle cannot retract.

A sliding block 6 is provided at the connection point of the first connecting rod 4 and the needle base 3. The barrel 1 includes a sliding barrel 7 to cooperate with the sliding block 6 and the sliding block 6 is capable of reciprocating linearly within the sliding barrel 7. A guiding function can be realized by cooperation between the sliding block 6 and the sliding barrel 7, which cause the first connecting rod 4 can only reciprocate linearly within the sliding barrel 7. Through the needle base 3 driving the needle body 2 to only reciprocate linearly, shaking can be avoided so that subsequent tattooing operation can be performed conveniently and correctly. In addition, a position limiting ring 8 is provided at the bottom of the sliding barrel 7. An end cover 9 is provided at the top of the sliding barrel 7. This end cover 9 is fitted over the first connecting rod 4 and located between the sliding block 6 and the first connecting part 5. The sliding block 6 reciprocates linearly between the position limiting ring 8 and the end cover 9. By cooperation between the position limiting ring 8 and the end cover 9, the sliding block 6 can only reciprocate linearly in between. That is to say, when the needle body 2 is extended, because the extending length of the needle body 2 is restricted by the position limiting ring 8, it can effectively prevent a receivers from being tattooed too deep.

In the non-resilient tattooing needle of the present invention, the barrel 1 further includes an ink chamber 10 and a needle nozzle 11. A sidewall of the ink chamber 10 is provided with an injection hole 12 and antislip stripes 13. The ink chamber 10 is connected to the sliding barrel 7. The needle nozzle 11 is of approximately tapered shape and includes a tapered mouth and a tapered tip. The tapered mouth is connected with the ink chamber 10. The needle body 2 reciprocates linearly inside the tapered tip and is capable of extending beyond or retracting within the tapered tip.

The antislip stripes 13 can facilitate holding operations of users. The tapered shape of the needle nozzle 11 can further restrict the movement range of the needle body 2, thereby preventing substantial shaking and side-to-side movement thereof and facilitating subsequent tattooing operations.

In some embodiments, the needle nozzle 11 is made of a transparent material(s). The advantageous effect of such a solution is that a viewer can visually know the internal conditions of the needle nozzle 11 conveniently, such as the ink level in the needle nozzle 11. The extending or retracting conditions of the needle body 2 can also be seen which can be useful for preventing jamming of the needle body 2.

The needle body 2 of the present invention can be implemented by various structures, such as a single needle body 2, a double-needle body 2 or a multiple-needle body 2. The specific structure of the needle body 2 can be selected based on the tattooing areas and positions.

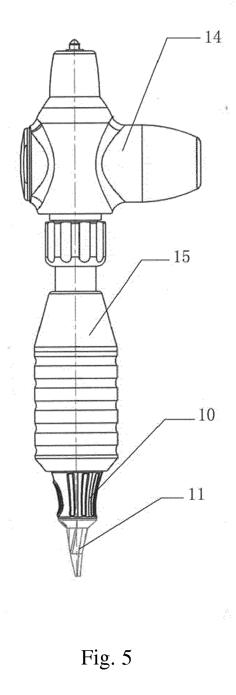

As shown in Figures 5 to 11, according to another aspect of the present invention, a tattooing device is provided which comprises of an electric motor 14, a handle housing 15, a second connecting rod 16 and an aforementioned tattooing needle. The electric motor 14 is located at the top of the handle housing 15. The second connecting rod 16 is arranged within the handle housing 15. The handle housing 15 is connected with the barrel 1. One end of the second connecting rod 16 is driven by the electric motor 14 and the other end thereof is provided with a second connecting part17. The second connecting part17 is to be rigidly connected to the first connecting part 5. The electric motor 14 drives the second connecting rod 16 to reciprocate linearly in the handle housing 15. The second connecting rod 16 in turn drives the first connecting rod 4 to move. And, the first connecting rod 4 drives the needle body 2 to reciprocate linearly in the barrel 1.

The needle of the tattooing device of the present invention does not employ any spring, rubber band or rubber ring to provide resilient force but it is just driven by an electric motor 14 directly. The electric motor 14 drives the second connecting rod 16 to reciprocate linearly in the handle housing 15. The second connecting rod 16 drives the first connecting rod 4 to move and the first connecting rod 4 in turn drives the needle body 2 to reciprocate linearly in the barrel 1. Because the second and first connecting parts 17, 5 are rigidly connected together, retraction of the needle of the present invention is achieved by driven of the electric motor 14 directly, which thereby avoids a delayed retraction and realizes instant retraction. Further, because extending and retraction of the needle of the present invention are driven by the electric motor 14, when pushing out the needle body 2, the electric motor 14 does not need to work against resilient forces. Therefore, both the current and power of the electric motor 14 can be kept stable and this thereby greatly prolongs the service life thereof.

The first connecting part 5 and the second connecting part 17 may be connected by way of hooking, clamping or buckling, which however renders the structure complex. A preferred connecting manner is by magnetic connection, which can be realized by using a magnet as one of the first and second connecting parts 5, 17 or using magnets for both of them. Usually, the needle is a disposable member. To save cost, the second connecting part 17 therefore is normally selected as a magnet and the first connecting part 5 is selected from a material that can be easily attracted by a magnet (such as iron). A similar effect can be realized by designing the first and second connecting parts to be able to connect via a hooking, clamping or buckling.

In the present invention, an output shaft 18 of the electric motor 14 is perpendicular to the second connecting rod 16 and it is connected with an eccentric shaft 19. The eccentric shaft 19 rotates circumferentially about the output shaft 18 of the electric motor 14 as the axis. The eccentric shaft 19 is connected with the second connecting rod 16. When rotating circumferentially, the eccentric shaft 19 drives the second connecting rod 16 to reciprocate linearly in the handle housing 15. By rotation of the electric motor 14 and provision of the eccentric shaft 19, the second connecting rod 16 can be driven to reciprocate linearly in the handle housing 15. The top of the second connecting rod 16 is provided with a connecting ring 20 equipped with a rubber ring 21. The eccentric shaft 19 is fitted in the rubber ring 21. Transmission of movement of the electric motor 14 and the second connecting rod 16 can be realized through cooperation among the eccentric shaft 19, the rubber ring 21 and the connecting ring 20. Rotation of the electric motor 14 can drive the second connecting rod 16 to reciprocate linearly in the handle housing 15. The rubber ring 21 can prevent abrasion of the connecting ring 20 and it can be replaced when worn.

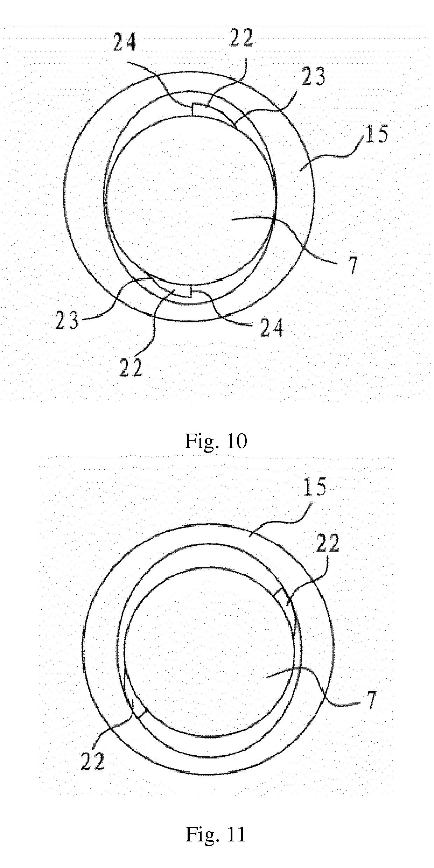

As shown in Figure 11, on outer wall of the sliding barrel 7 of the barrel 1 is provided with two or more clamping edges 22 usually disposed symmetrically. One side part of the clamping edges 22 is a connecting area 23 and the other side part thereof is a protruding part 24. The connecting area 23 is in smooth connection with an external surface of the sliding barrel 7. A height difference is preset between the protruding part 24 and the external surface of the sliding barrel 7. One end of the handle housing 15 to be connected with the barrel 1 is of an elliptical shape.

As shown in Figure 11, when connecting the sliding barrel 7 of the barrel 1 with the handle housing 15, the sliding barrel 7 is inserted into the handle housing 15 in accordance with the long axis of the elliptical-shaped handle housing 15. As shown in Figure 12, by rotating the barrel 7 in a direction from the protruding part 24 towards the connecting area 23 until the clamping edges 22 are in a close contact with the inner wall of the elliptical-shaped handle housing 15 along its short axis direction, a firm contact between the barrel 7 and the handle housing 15 is achieved.

The above is only some embodiments of the present invention. Various modifications and improvements may be made by those skilled in the art without departing from the inventive concept of the present invention and still fall into the protection scope of the present invention.

## Claims

1. A non- resilient tattooing needle comprising:
a barrel (1);
a needle body (2) disposed inside the barrel (1);
a needle base (3) for fixing the needle body (2);
a first connecting rod (4) connecting with the needle base (3) and arranged along a same axis with the needle body (2); and
a first connecting part (5) disposed at the top of the first connecting rod (4),
wherein the first connecting part (5) reciprocates under an external force and thus drives the needle body (2), via the first connecting rod (4) and the needle base (3), to reciprocate linearly in the barrel (1).

2. The non-resilient tattooing needle according to claim 1, wherein
a sliding block (6) is connected in-between the first connecting rod (4) and the needle base (3),
the barrel (1) includes a sliding barrel (7) to cooperate with the sliding block (6), and
the sliding block (6) is capable of reciprocating linearly within the sliding barrel (7).

3. The non- resilient tattooing needle according to claim 2, wherein
a position limiting ring (8) is provided at the bottom of the sliding barrel (7),
an end cover (9) is provided at the top of the sliding barrel (7),
the end cover (9) is fitted over the first connecting rod (4) and disposed between the sliding block (6) and the first connecting part (5), and
the sliding block (6) reciprocates linearly between the position limiting ring (8) and the end cover (9).

4. The non- resilient tattooing needle according to claim 3, wherein
the barrel (1) further includes an ink chamber (10) and a needle nozzle (11),
an injection hole (12) and antislip stripes (13) are provided on sidewall of the ink chamber (10),
the ink chamber (10) is connected with the sliding barrel (7),
the needle nozzle (11) is of approximately tapered shape and comprises a tapered mouth and a tapered tip,
the tapered mouth is in communication with the ink chamber (10), and
the needle body (2) reciprocates linearly with regard to the tapered tip and is capable of extending beyond or retracting within the tapered tip.

5. The non- resilient tattooing needle according to claim 4, wherein the needle nozzle (11) is made of transparent materials.

6. A tattooing device comprising:
an electric motor (14),
a handle housing (15),
a second connecting rod (16) and
a tattooing needle according to claims 1,
wherein the electric motor (14) is located at the top of the handle housing (15),
the second connecting rod (16) is arranged within the handle housing (15),
the handle housing (15) is to be connected with the barrel (1),
one end of the second connecting rod (16) is driven by the electric motor (14) and the other end of the second connecting rod (16) is provided with a second connecting part (17),
the second connecting part (17) is to be rigidly connected with the first connecting part (5),
the electric motor (14) drives the second connecting rod (16) to reciprocate linearly in the handle housing (15),
the second connecting rod (16) thus drives the first connecting rod (4) to move, and
the first connecting rod (4) drives the needle body (2) to reciprocate linearly in the barrel (1).

7. The tattooing device according to claim 6, wherein the first connecting part (5) and/or the second connecting part (17) is/are a magnet/magnets.

8. The tattooing device according to claim 6, wherein
an output shaft (18) of the electric motor (14) is perpendicular to the second connecting rod (16) and is connected with an eccentric shaft (19),
the eccentric shaft (19) rotates circumferentially about the output shaft (18) of the electric motor (14) as the axis,
the eccentric shaft (19) is connected with the second connecting rod (16), and
when rotating circumferentially, the eccentric shaft (19) drives the second connecting rod (16) to reciprocate linearly in the handle housing (15).

9. The tattooing device according to claim 8, wherein
the top of the second connecting rod (16) is provided with a connecting ring (20) equipped with a rubber ring (21), and
the eccentric shaft (19) is fitted in the rubber ring (21).

10. The tattooing device according to claim 6, wherein
two symmetrically disposed clamping edges (22) are provided on outer wall of the sliding barrel (7) of the barrel (1),
one side part of the clamping edges (22) is a connecting area (23) and the other side part thereof is a protruding part (24),
the connecting area (23) is in smooth connection with the outer wall of the sliding barrel (7),
a height difference exists between the protruding part (24) and the surface of the outer wall of the sliding barrel (7), and
one end of the handle housing (15) to be connected with the barrel (1) is of an elliptical shape.
